# EUROPEAN PATENT APPLICATION

(11) **EP 1 156 035 A2**
(43) Date of publication of application: **21.11.2001**
(21) Application number: 01121707.2
(22) Date of filing: 11.12.1998
(51) Int. Cl.: C07C 303/32, C07C 309/42, C07C 67/08, C07C 67/14, C07F 9/12

(54) **Preparation of esters**

(30) Priority: 11.12.1997 GB 9726246
(62) Divisional of application: 98310193.2
(71) Applicant: The Associated Octel Company Limited, Manchester M90 4AA (GB)
(72) Inventor: Radley, Peter Michael, Research & Development Dept, Oil Sites Road, Ellesmere Port CH65 4HF (GB); Head, Robert Arthur, Research & Development Dept, Oil Sites Road, Ellesmere Port CH65 4HF (GB); Small, Andrew, Research & Development Dept, Oil Sites Road, Ellesmere Port CH65 4HF (GB)
(74) Representative: Matthews, Derek Peter

(57) **Abstract**

A process for the preparation of phenol esters which have utility as bleach activators.

## Description

The invention relates to an improved process for the preparation of phenol esters which have utility as bleach activators.

As a result of renewed interest in the use of phenolsulphonate esters as bleach activators, there has been a resurgence of interest in improved methods of producing them.

EP 148148 describes the reaction of anhydrous sodium phenol sulphonate with an alkanoyl halide at 90-200°C in the absence of a solvent.

EP 120591 describes a similar reaction but uses refluxing halocarbon solvents as the reaction medium. Additionally, 1.2-1.5 mole equivalents of alkanoyl halide are employed as are extended reaction times (20 hours) to effect the reaction.

EP 150532 describes the reaction of an acyl chloride with anhydrous disodium phenol sulphonate (^{∼} 1:1 mole ratio) in glyme as a solvent. Typical reaction yields are 60-80%.

EP 166571 describes a similar process but uses a 1.2-1.5 mole equivalent of acyl chloride to give products in isolated yields of 40-65%.

Improved yields are claimed in EP 220826 wherein certain linear acyl chlorides are reacted with sodium phenol sulphonate at 160-170°C over 3-4 hours. A 2:1 mole ratio is typically employed and the necessary recycle of unreacted acid chloride is demonstrated.

Process improvements are also claimed in EP 402048 wherein a phase transfer catalyst is used to effect the reaction of an acid chloride with a phenol sulphonate salt. Isolated yields are typically 60-70% based on the starting acid chloride employed.

EP 164786 describes the use of potassium salts of phenolsulphonic acid instead of the sodium salt to improve reaction yields although no quantitative data is provided.

The prior art discussed above clearly demonstrates that obtaining conversion factors greater than 70% using acyl chlorides and salts of phenol sulphonic acid is difficult to achieve. Thus, a number of alternative processes have also been taught.

EP 125641 teaches the use of transesterification to effect the desired reaction whereby phenol esters of aliphatic acids are reacted with hydroxybenzene sulphonate salts. Very large molar excesses of phenol esters are used (up to 5:1) resulting in isolated yields of only 20-30% based on the starting phenol ester employed.

US 4544503 describes the reaction of phenol sulphonate salts with CO and an olefin at >125°C and >500 psig in an inert atmosphere with a metal carbonyl catalyst. Moderate yields are given (^{~}60%), and products are highly coloured.

Sulphonation of an appropriate phenol ester is described in EP 165480 and EP 201222 wherein good yields (70-90%) are claimed by careful control of the SO₃ sulphonation conditions.

Alternatively, the use of a mixed anhydride using acetic anhydride is described in US 4735740.

Similarly, the preparation of 6-benzoylamidocaproyl oxybenzene sulphonate is described in US 4634551 whereby the acid is reacted with anhydrous sodium phenol sulphonate using trifluoroacetic anhydride. Isolated yield was 48%.

EP 153222 describes the use of a symmetric anhydride to effect the reaction, acetic anhydride is disclosed but this process suffers from the principal disadvantage of only giving a maximum yield of 50% based on the starting acid on a first-pass process. Recycling is necessary and this is described.

Finally, the use of the Schotten-Baumann technique in the preparation of esters of oxybenzene sulphonate salts is given in EP 294073. The only synthesis exemplified involves the reaction of sodium phenol sulphonate with benzoyl chloride to make sodium benzoyloxybenzene sulphonate. No quantitative data is provided.

Summarising these disclosures, it can be seen that, despite considerable prior art teaching, there remain significant problems in finding simple, high-yielding, low colour generating, cost-effective processes to make oxybenzene salts such as oxybenzene sulphonate salts.

We have now developed improved processes for the preparation of phenol esters of formula (I) 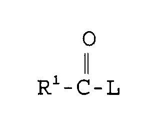 wherein R¹ is a linear or branched alkyl or alkenyl group of 3 to 20 carbon atoms optionally interrupted by -O- or -NH- and optionally substituted by an oxo group and L is a group of formula 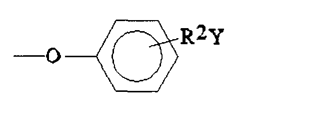 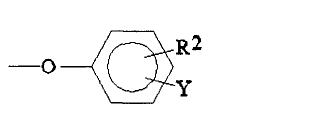 or 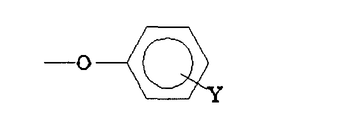 in which R² is an alkyl group or alkylene chain containing 1 to 8 carbon atoms and Y is a group -CO₂^{⊖}M^{⊕}, -SO₃^{⊖}M^{⊕} or -PO₄^{⊖}M^{⊕} in which M^{⊕} is a cation, preferably an alkali metal, ammonium or substituted ammonium cation. In particular M^{⊕} may be a sodium or potassium cation. It will be appreciated that the -O- or -NH- in R¹ can be adjacent to the oxo substituent thus forming an ester or amide linkage.

In a first aspect of the invention a carboxylic acid corresponding to R¹-CO- as defined above is reacted with a substituted phenol corresponding to L as defined above in the presence of a chlorinating agent such as thionyl chloride, phosgene, POCl₃ or PCl₅. At the end of the reaction the desired phenol ester, formed in high yield, is obtained by filtration. This then comprises a "one pot" synthesis of phenol ester from carboxylic acid.

The term "chlorinating agent" as used above means an agent capable of forming an acyl chloride from a carboxylic acid.

In a second aspect of the invention an acyl chloride corresponding to R¹-CO- as defined above is reacted with a substituted phenol as defined above in the presence of a tertiary amine or tertiary amide catalyst. At the end of the reaction the desired phenol ester, formed in high yield, is obtained by filtration.

Tertiary amines and amides useful as catalysts include dimethylformamide, triethylamine, pyridine and 4-dimethylaminopyridine. The acyl chloride corresponding to R¹-CO- may be prepared by reaction of a carboxylic acid corresponding to R¹-CO- with a chlorinating agent as defined above, for example thionyl chloride.

Suitable solvents for use in both aspects of the invention are aromatic hydrocarbons, cycloalkanes, dialkylamides, cyclic dialkylamides, ethers, alkyl nitriles, dialkylketones and esters.

Substituted phenols corresponding to L as defined above are commercially available from numerous suppliers. It is important that any water present is partly or fully removed before use. This can be achieved by drying or by azeotropic removal of the water in the presence of an appropriate solvent. In such cases for best results a reaction solvent should be selected which permits the azeotropic removal of water prior to the addition of the chlorinating agent. Examples of such solvents include aliphatic and aromatic hydrocarbons, ketones, cyanoalkanes and dialkylamides, the selection of which is well within the knowledge of those skilled in the art.

Suitable reaction temperatures for both the "one pot" and catalysed reactions are -20°C to 250°C. Preferably the "one pot" reaction is carried out above 60°C to allow HCl and SO₂ to off-gas. In the preparation of the acyl chloride for the catalysed reaction it is also convenient to raise the temperature to allow HCl and SO₂ to off-gas.

Reaction time for both reactions is variable to completion (dependent upon solvent system and temperature). Generally the reaction time is less than 5 hours and more than 1 hour.

The colour of the solid product is a commercially important attribute. Material of a white appearance is desired. The colour of the product can be quantified in terms of Hunter colour values L, a and b, where colour improvement is shown by an increase in L and a decrease in a and b. The colour characteristics of product from this process can be improved by addition of a polar solvent to the final reaction mixture. A suitable solvent is acetone. Suitable quantities will need to be determined on a case-by-case basis, but will generally lie in the range 5-50% by volume, relative to the reaction volume.

Whilst it is normally convenient to react the substituted phenol and the carboxylic acid and chlorinating agent, or the substituted phenol and the acyl chloride, in approximately equimolar amounts, it will occasionally be found that the reactions will not proceed to completion under these conditions, for example if the substituted phenol contains a small amount of residual water. An additional quantity of chlorinating agent may then be added to bring the reaction to completion.

The invention is particularly applicable to the synthesis of sodium (6-nonamidohexanoyl)oxybenzene sulphonate.

### Example 1

### One-pot preparation of sodium (6-nonamidohexanoyl)oxybenzene sulphonate

A slurry of 23.2g (0.10 mol) of sodium phenolsulphonate dihydrate and 250 ml of toluene was heated to reflux for three hours with azeotropic removal of a total of 30 ml of toluene and water using a Dean-Stark trap. The mixture was allowed to cool to 20°C and 27.1g (0.10 mol) of nonamidohexanoic acid added. The mixture was cooled to 0-5°C and 12.0g (0.10 mol) of thionyl chloride added. The mixture was gently heated to reflux for three hours and then allowed to cool to room temperature. The white solid product was collected by filtration, washed with 20 ml of toluene and dried at 40-50°C under reduced pressure to give the sodium (6-nonamidohexanoyl)-oxybenzene sulphonate, 44.1g (0.098 mol), 98% yield, 96.35% assay by HPLC.

### Example 2

### Preparation of sodium (6-nonamidohexanoyl)oxybenzene sulphonate via the acid chloride of nonamidohexanoic acid

To a slurry of 20.0g (0.074 mol) of nonamidohexanoic acid in 40 ml of toluene at 20-25°C was added 8.9g (0.074 mol) of thionyl chloride. The mixture was stirred at this temperature for one hour before heating to 75-80°C for 30 minutes whilst purging the mixture with nitrogen gas to assist in the removal of SO₂ and HCl gases.

In a separate vessel a slurry of 17.2g (0.074 mol) of sodium phenolsulphonate dihydrate in 100 ml of toluene was heated to reflux for three hours with azeotropic removal of water using a Dean-Stark trap. The mixture was cooled to room temperature and 0.5g (0.007 mol) of dimethylformamide and the toluene solution of nonamidohexanoyl chloride added. The mixture was then heated to reflux for 17 hours before cooling to room temperature. The white solid product was collected by filtration, washed with 20 ml of toluene and dried at 40-50°C under reduced pressure to give the sodium (6-nonamidohexanoyl)oxybenzene-sulphonate, 32.9g (0.073 mol), 99% yield with assay of 92.5%.

### Example 3

### Use of acetone to improve colour characteristics

Two procedures identical to Example 1 were carried out. One was worked up as described in Example 1. To the other 100 ml of acetone were added at room temperature after the reaction phase had finished, followed by filtration as in Example 1. The characteristics of the products were:

| | No added acetone | Added acetone |
|---|---|---|
| Yield (%) | 93.1 | 99.6 |
| Assay (%) | 94 | 94 |
| Hunter L | 83.6 | 88.8 |
| a | 0.88 | 0.51 |
| b | 8.63 | 4.39 |

## Claims

1. A process for preparing a phenol ester of formula I 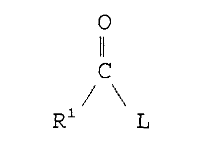 (wherein R¹ is a linear or branched alkyl or alkenyl group optionally interrupted by an -O- or -NH- group and optionally substituted by an oxo group and L is a group of formula 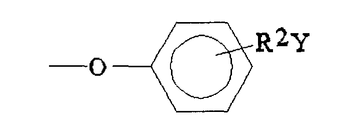 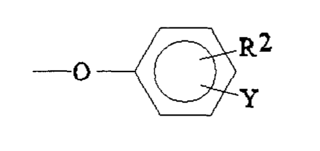 or 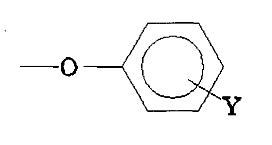 in which R² is an alkyl group or alkylene chain and Y is a group -CO₂⁻M⁺, SO₃⁻M⁺ or PO₄⁻M⁺ (wherein M is a cation)) comprising the step of reacting a substituted phenol corresponding to L with an acyl chloride corresponding to R¹CO- in the presence of a tertiary amine or tertiary amide catalyst.

2. A process as claimed in claim 1 wherein said substituted phenol is subjected to removal of water prior to said reaction step.

3. A process as claimed in claim 2 wherein said removal of water is carried out azeotropically in an appropriate solvent.

4. A process as claimed in any preceding claim in which the said reaction step is carried out under reflux.

5. A process as claimed in claim 4 in which the said reaction step is carried out at a temperature above 60°C.

6. A process as claimed in any preceding claim wherein said catalyst is dimethylformamide.

7. A process as claimed in any preceding claim wherein R¹CO- is a 6-acylamidohexanoyl group.

8. A process as claimed in any preceding claim further comprising addition of a colour-improving amount of a polar solvent after said reaction step, and recovery of said phenol ester by filtration.

9. A process as claimed in claim 8 wherein said polar solvent is acetone.

10. A process as claimed in claim 1 in which a quantity of chlorinating agent is added to bring the reaction to completion.

11. A process as claimed in any preceding claim wherein said compound of formula I is sodium 6-(nonamidohexanoyl)-oxybenzene sulphonate.
